# EUROPÄISCHE PATENTSCHRIFT

(11) **EP 1 028 703 B1**
(45) Veröffentlichungstag und Bekanntmachung des Hinweises auf die Patenterteilung: **11.09.2002**
(21) Anmeldenummer: 98956899.3
(22) Anmeldetag: 30.10.1998
(51) Int. Cl.: A61K 7/32

(54) **WÄSSRIGE KOSMETISCHE ZUBEREITUNGEN IN STIFTFORM**
AQUEOUS COSMETIC PREPARATIONS IN STICK FORM
PREPARATIONS COSMETIQUES AQUEUSES SOUS FORME DE STICK

(30) Priorität: 11.11.1997 DE 19749819
(43) Veröffentlichungstag der Anmeldung: 23.08.2000
(73) Patentinhaber: Cognis Deutschland GmbH & Co. KG, 40589 Düsseldorf (DE)
(72) Erfinder: GUCKENBIEHL, Bernhard, D-50858 Köln (DE); BANOWSKI, Bernhard, D-40597 Düsseldorf (DE); TESMANN, Holger, D-41363 Jüchen (DE)
(86) Internationale Anmeldenummer: EP9806896
(87) Internationale Veröffentlichungsnummer: WO99023866

(56) Entgegenhaltungen:
- DE-A- 19 530 220

## Beschreibung

### Gebiet der Erfindung

Die Erfindung betrifft wäßrige, seifen- und wachsfreie kosmetische Stiftpräparate, enthaltend Glucoside, Ölkörper und ausgewählte Emulgatoren sowie die Verwendung der Mischungen zur Herstellung von Deostiften.

### Stand der Technik

Wasserhaltige kosmetische Stiftpräparate, die als Antitranspirant- oder Deodorantprodukte auf dem Markt zu finden sind, enthalten vornehmlich Seife (Natriumstearat), Ölkörper und Bakterizide. Sie weisen einen alkalischen pH-Wert von ca. 9 auf. Als nachteilig wird vom Verbraucher das mit diesen Stiften verbundene seifige Hautgefühl angesehen. Eine neuere Entwicklung betrifft Stifte, die bekannte Antitranspirantwirkstoffe, wie z.B. Aluminiumchlorhydrat (ACH) enthalten. Sie müssen bei einem sauren pH-Wert von ca. 4 formuliert werden und benötigen dazu besondere Verdickersysteme, wie z.B. Polydiole in Kombination mit Dibenzylidensorbitol. Daneben gibt es seit vielen Jahren eine Vielzahl von Antitranspirantstiften auf Basis natürlicher oder synthetischer Wachse auf dem Markt, in denen der Wirkstoff als Puder in die Wachsmatrix eingebracht wird. Hierbei ist von Nachteil, daß die Stifte stark fettend sind und häufig ein weißer Rückstand auf der Haut verbleibt.

Die komplexe Aufgabe der vorliegenden Erfindung hat daher darin bestanden, Stiftpräparate zur Verfügung zu stellen, die frei von den geschilderten Nachteilen sind. Insbesondere sollten die Stifte frei von Seifen sein, so daß saure Wirkstoffe eingearbeitet werden können und auch keine natürlichen Wachse enthalten, um Rückstände auf der Haut zu vermeiden. Gleichzeitig sollten sich die Stifte durch ein verbessertes Hautgefühl, hohe Konsistenz und Temperaturbeständigkeit sowie Transparenz bzw. Weißgrad auszeichnen.

### Beschreibung der Erfindung

Gegenstand der Erfindung sind wäßrige kosmetische Zubereitungen in Stiftform, enthaltend
(a) Alkyl- und/oder Alkenyloligoglykoside,
(b) Ölkörper und
(c) nichtionische Emulgatoren,
mit der Maßgabe, daß die Zubereitungen frei sind von Seifen und natürlichen Wachsen.

Überraschenderweise wurde gefunden, daß die erfindungsgemäßen Zubereitungen nicht nur eine ausreichend hohe Konsistenz und Temperaturbeständigkeit zeigen, sondern auch ein vorteilhaftes Hautgefühl vermitteln. Die Zubereitungen sind seifenfrei und erlauben daher die Einarbeitung saurer Wirkstoffe, wie z.B. Aluminiumchlorhydrat. Hierbei schließt die Erfindung die Erkenntnis mit ein, daß bei der Formulierung keine Wirkstoffpulver, sondern ohne weiteres auch wäßrige Lösungen eingesetzt werden können, was die Herstellung und die homogene Verteilung im Stift erheblich vereinfacht. Die Stifte sind transparent gelig oder rein weiß und hinterlassen bei der Anwendung keine störenden Rückstände.

### Alkyl- und/oder Alkenyloligoglykoside

Alkyl- und Alkenyloligoglykoside stellen bekannte nichtionische Tenside dar, die der Formel **(I)** folgen,

**R**^{**1**}**O-[G]**_{**p**} (I)

in der R¹ für einen Alkyl- und/oder Alkenylrest mit 8 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie, beispielsweise durch sauer katalysierte Acetalisierung von Glucose mit Fettalkoholen erhalten werden.

Die Alkyl- und/oder Alkenyloligoglykoside können sich von Aldosen bzw. Ketosen mit 5 oder 6 Kohlenstoffatomen, vorzugsweise der Glucose ableiten. Die bevorzugten Alkyl- und/oder Alkenyloligoglykoside sind somit Alkyl- und/oder Alkenyloligo**glucoside.** Die Indexzahl p in der allgemeinen Formel **(I)** gibt den Oligomerisierungsgrad (DP), d. h. die Verteilung von Mono- und Oligoglykosiden an und steht für eine Zahl zwischen 1 und 10. Während p in einer gegebenen Verbindung stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt. Der Alkyl- bzw. Alkenylrest R¹ kann sich von primären Alkoholen mit 8 bis 22, vorzugsweise 12 bis 16 Kohlenstoffatomen ableiten. Typische Beispiele sind Octanol, Decanol, Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkyloligoglucoside auf Basis von Lauryl- und/oder Myristylalkohol sowie entsprechender technischer Kokosfettalkoholschnitte mit einem DP im Bereich von 1 bis 3. Der Anteil der Glucoside an den Zubereitungen kann 1 bis 40 und vorzugsweise 3 bis 30 Gew.-% betragen.

### Ölkörper

Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren, insbesondere C₆-C₂₀, mit linearen C₆-C₂₂-Fettalkoholen, insbesondere C₆-C₂₀, Ester von verzweigten C₆-C₁₃-Carbonsäuren mit linearen C₆-C₂₂-Fettalkoholen, insbesondere C₆-C₂₀, Ester von linearen C₆-C₂₂-Fettsäuren, insbesondere C₆-C₁₈, mit verzweigten Alkoholen, insbesondere 2-Ethylhexanol, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure Dicarbonsäureester, insbesondere, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare C₆-C₂₂-Fettalkoholcarbonate, Guerbetcarbonate, Ester der Benzoesäure mit linearen und/oder verzweigten C₆-C₂₂-Alkoholen (z.B. Finsolv® TN), Dialkylether, Ringöffnungsprodukte von epoxidierten Fettsäureestern mit Polyolen, Siliconöle und/oder aliphatische bzw. naphthenische Kohlenwasserstoffe in Betracht. Der Anteil der Ölkörper an den Zubereitungen kann 1 bis 50 und vorzugsweise 3 bis 30 Gew.-% betragen.

### Nichtionische Emulgatoren

Als nichtionische Emulgatoren kommen beispielsweise Niotenside aus mindestens einer der folgenden Gruppen in Frage:
(c1) Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
(c2) C_{12/18}-Fettsäuremono- und -diester von Anlagerungsprodukten von 1 bis 30 Mol Ethylenoxid an Glycerin;
(c3) Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
(c4) Anlagerungsprodukte von 1, insbesondere 15, bis 60 Mol Ethylenoxid an Ricinusöl und/oder gehärtetes Ricinusöl;
(c5) Polyol- und insbesondere Polyglycerinester wie z.B. Polyglycerinpolyricinoleat oder Polyglycerinpoly-12-hydroxystearat. Ebenfalls geeignet sind Gemische von Verbindungen aus mehreren dieser Substanzklassen;
(c6) Anlagerungsprodukte von 2 bis 15 Mol Ethylenoxid an Ricinusöl und/oder gehärtetes Ricinusöl;
(c7) Partialester auf Basis linearer, verzweigter, ungesättigter bzw. gesättigter C_{12/22}-Fettsäuren, Ricinolsäure sowie 12-Hydroxystearinsäure und Glycerin, Polyglycerin, Pentaerythrit, Dipentaerythrit, Zuckeralkohole (z.B. Sorbit), Alkylglucoside (z.B. Methylglucosid, Butylglucosid, Laurylglucosid) sowie Polyglucoside (z.B. Cellulose);
(c8) Mono-, Di- und/oder Trialkylphosphate;
(c9) Wollwachsalkohole;
(c10) Polysiloxan-Polyalkyl-Polyether-Copolymere bzw. entsprechende Derivate;
(c11) Mischester aus Pentaerythrit, Fettsäuren, Citronensäure und Fettalkohol gemäß **DE-PS 1165574** und/oder Mischester von Fettsäuren mit 6 bis 22 Kohlenstoffatomen, Methylglucose und Polyolen, vorzugsweise Glycerin sowie
(c12) Polyalkylenglycole.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE-PS 2024051** als Rückfettungsmittel für kosmetische Zubereitungen bekannt. Im Sinne der Erfindung ist die Verwendung von Polygycerinpoly-12-hydroxystearaten und/oder Polyglycerinpolyricinoleaten gemäß **DE-A1 4420516** (Henkel) bevorzugt, da das Ölbindevermögen dieser Emulgatoren das der üblicherweise eingesetzten Glycerinmonoricinoleate bzw. des Ricinusöls deutlich übertrifft. Der Anteil der nichtionischen Emulgatoren an den Zubereitungen kann 1 bis 30 und vorzugsweise 3 bis 10 Gew.-% betragen. Vorzugsweise werden lipophile Emulgatoren mit HLB-Werten unterhalb von 10 eingesetzt.

### Co-Emulgatoren

Als Co-Emulgatoren kommen anionische und/oder amphotere bzw. zwitterionische Tenside in Frage. Typische Beispiele für anionische Tenside sind Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Hydroxymischethersulfate, Monoglycerid(ether)-sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für amphotere bzw. zwitterionische Tenside sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten beispielsweise **J.Falbe (ed.), "Surfactants in Consumer Products", Springer Verlag, Berlin, 1987, S. 54-124** oder **J.Falbe (ed.), "Katalysatoren, Tenside und Mineralöladditive", Thieme Verlag, Stuttgart, 1978, S. 123-217** verwiesen. Der Anteil der Co-Emulgatoren an den Zubereitungen kann 0 bis 30 und vorzugsweise 1 bis 15 Gew.-% betragen.

### Konsistenzgeber

Als zusätzliche Komponente mit co-emulgierenden Eigenschaften können die Zubereitungen weiterhin Konsistenzgeber beispielsweise vom Typ der **Fettalkohole** enthalten. Hierunter sind primäre aliphatische Alkohole der Formel **(II)** zu verstehen,

**R**^{**2**}**OH** (II)

in der R² für einen aliphatischen, linearen oder verzweigten Kohlenwasserstoffrest mit 6 bis 22 Kohlenstoffatomen und 0 und/oder 1, 2 oder 3 Doppelbindungen steht. Typische Beispiele sind Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestem auf Basis von Fetten und Ölen oder Aldehyden aus der Roelen'schen Oxosynthese sowie als Monomerfraktion bei der Dimerisierung von ungesättigten Fettalkoholen anfallen. Bevorzugt sind technische Fettalkohole mit 12 bis 18 Kohlenstoffatomen wie beispielsweise Kokosfettalkohol. Der Anteil der Fettalkohole an den Zubereitungen kann 1 bis 35 und vorzugsweise 5 bis 30 Gew.-% betragen.

Alternativ können als Konsistenzgeber auch **Diole** eingesetzt werden. Typische Beispiele hierfür sind 1,12-Dodecandiol, 1,16-Hexadecandiol, 12-Hydroxystearylalkohol sowie Ringöffnungsprodukte von epoxidierten C₆-C₂₂-Olefinen mit Wasser oder Polyolen, vorzugsweise Glycerin. Der Anteil der Diole an den Zubereitungen kann 1 bis 35 und vorzugsweise 5 bis 30 Gew.-% betragen.

Als weitere Gruppe von Konsistenzgebem können auch **Fettsäurepartialglyceride** eingesetzt werden, also Monoglyceride, Diglyceride und deren technische Gemische, die herstellungsbedingt noch geringe Mengen Triglyceride enthalten können. Die Partialglyceride folgen vorzugsweise der Formel **(III)**, 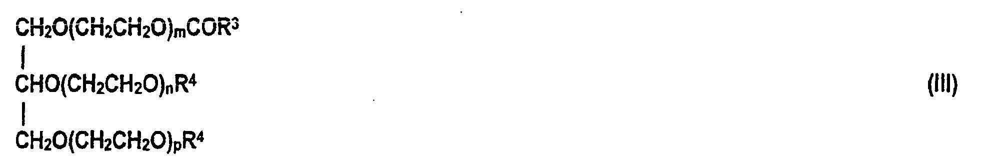 in der R³CO für einen linearen oder verzweigten, gesättigten und/oder ungesättigten Acylrest mit 6 bis 22, vorzugsweise 12 bis 18 Kohlenstoffatomen, R⁴ und R⁵ unabhängig voneinander für R¹CO oder OH und die Summe (m+n+p) für 0 oder Zahlen von 1 bis 100, vorzugsweise 5 bis 25 steht, mit der Maßgabe, daß mindestens einer der beiden Reste R⁴ und R⁵ OH bedeutet. Typische Beispiele sind Monound/oder Diglyceride auf Basis von Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Vorzugsweise werden technische Laurinsäureglyceride, Palmitinsäureglyceride, Stearinsäureglyceride, Isostearinsäureglyceride, Ölsäureglyceride, Behensäureglyceride und/oder Erucasäureglyceride eingesetzt, welche einen Monoglyceridanteil im Bereich von 50 bis 95, vorzugsweise 60 bis 90 Gew.-% aufweisen. Der Anteil der Fettsäurepartialglyceride an den Zubereitungen kann 1 bis 35 und vorzugsweise 5 bis 30 Gew.-% betragen.

### Wirkstoffe

Als **Antitranspirantien** kommen z.B. Aluminiumchlorhydate in Frage. Hierbei handelt es sich um farblose, hygroskopische Kristalle, die an der Luft leicht zerfließen und beim Eindampfen wäßriger Aluminiumchloridlösungen anfallen. Aluminiumchlorhydrat wird zur Herstellung von schweißhemmenden und desodorierenden Zubereitungen eingesetzt und wirkt wahrscheinlich über den partiellen Verschluß der Schweißdrüsen durch Eiweiß- und/oder Polysaccharidfällung [vgl. **J.Soc.Cosm. Chem. 24, 281 (1973)**]. Unter der Marke Locron® der Hoechst AG, Frankfurt/FRG, befindet beispielsweise sich ein Aluminiumchlorhydrat im Handel, das der Formel [Al₂(OH)₅Cl]*2,5 H₂O entspricht und dessen Einsatz besonders bevorzugt ist [vgl. **J.Pharm.Pharmacol. 26, 531(1975)].** Neben den Chlorhydraten können auch Aluminiumhydroxylactate sowie saure Aluminium/Zirkoniumsalze eingesetzt werden. Als weitere Deowirkstoffe können **Esteraseinhibitoren** zugesetzt werden. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen® CAT, Henkel KGaA, Düsseldorf/FRG). Die Stoffe inhibieren die Enzymaktivität und reduzieren dadurch die Geruchsbildung. Wahrscheinlich wird dabei durch die Spaltung des Citronensäureesters die freie Säure freigesetzt, die den pH-Wert auf der Haut soweit absenkt, daß dadurch die Enzyme inhibiert werden. Weitere Stoffe, die als Esteraseinhibitoren in Betracht kommen, sind Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbnonsäuren und deren Ester wie beispielsweise Citronensäure, Äpfelsäure, Weinsäure oder Weinsäurediethylester. **Antibakterielle Wirkstoffe,** die die Keimflora beeinflussen und schweißzersetzende Bakterien abtöten bzw, in ihrem Wachstum hemmen, können ebenfalls in den Stiftzubereitungen enthalten sein. Beispiele hierfür sind Chitosan, Phenoxyethanol und Chlorhexidingluconat. Besonders wirkungsvoll hat sich auch 5-Chlor-2-(2,4-dichlorphenoxy)-phenol erwiesen, das unter der Marke Irgasan® von der Ciba-Geigy, Basel/CH vertrieben wird. Der Anteil der Wirkstoffe an den Zubereitungen kann 0 bis 30 und vorzugsweise 0,1 bis 15 Gew.-% betragen.

### Siliconverbindungen

Geeignete Siliconverbindungen sind beispielsweise Dimethylpolysiloxane, Methylphenylpolysiloxane, cyclische Silicone sowie amino-, fettsäure-, alkohol-, polyether-, epoxy-, fluor- glucosid- und/oder alkylmodifizierte Siliconverbindungen, die bei Raumtemperatur sowohl flüssig als auch harzförmig vorliegen können. Der Anteil der Siliconverbindungen an den Zubereitungen kann 0 bis 20 und vorzugsweise 1 bis 10 Gew.-% betragen.

### Gewerbliche Anwendbarkeit

In einer bevorzugten Ausführungsform der Erfindung weisen die kosmetischen Zubereitungen die folgenden Zusammensetzungen auf:
(a) 1 bis 40, vorzugsweise 3 bis 30 Gew.-% Alkyl- und/oder Alkenyloligoglykoside,
(b) 10 bis 50, vorzugsweise 15 bis 35 Gew.-% Ölkörper,
(c) 1 bis 30, vorzugsweise 3 bis 10 Gew.-% nichtionische Emulgatoren,
(d) 0 bis 35, vorzugsweise 1 bis 15 Gew.-% Co-Emulgatoren,
(e) 0 bis 35, vorzugsweise 5 bis 30 Gew.-% Konsistenzgeber,
(f) 0 bis 30, vorzugsweise 1 bis 15 Gew.-% Wirkstoffe,
(g) 0 bis 20, vorzugsweise 1 bis 10 Gew.-% Siliconverbindungen sowie
(h) 0 bis 20, vorzugsweise 5 bis 10 Gew.-% niedere Alkohole,
mit der Maßgabe, daß sich die Angaben mit Wasser und gegebenenfalls weiteren üblichen Hilfs- und Zusatzstorfen zu 100 Gew.-% addieren. Der Wasseranteil in den Zubereitungen kann dabei 10 bis 70 und vorzugsweise 25 bis 50 Gew.-% betragen.

Als weitere Hilfs- und Zusatzstorfe können die erfindungsgemäßen Zubereitungen Überfettungsmittel enthalten, wie beispielsweise polyethoxylierte oder acylierte Lanolin- und Lecithinderivate, Polyolfettsäureester, Monoglyceride und Fettsäurealkanolamide. Als Lösemittel können neben Wasser niedere Alkohole, vorzugsweise Ethanol, sowie Polyole, wie z.B. Propylenglycol, Glycerin, Polyglycerin und dergleichen in Mengen bis zu 25 Gew.-% eingesetzt werden. Als Stabilisatoren können Metallsalze von Fettsäuren wie z.B. Magnesium-, Aluminium- und/oder Zinkstearat verwendet werden. Als biogene Wirkstoffe kommen beispielsweise in Frage Bisabolol, Allantoin, Phytantriol, Panthenol, AHA-Säuren, Tocopherole, Pflanzenextrakte und Vitaminkomplexe. Als Konservierungsmittel können Parabene oder Phenoxyethanol, gegebenenfalls in Abmischung mit Ethanol, eingesetzt werden. Als Farbstoffe können die für kosmetische Zwecke geeigneten und zugelassenen Substanzen verwendet werden, wie sie beispielsweise in der Publikation "Kosmetische Färbemittel" der Farbstoffkommission der Deutschen Forschungsgemeinschaft, Verlag Chemie, Weinheim, 1984, S.81-106 zusammengestellt sind. Diese Farbstoffe werden üblicherweise in Konzentrationen von 0,001 bis 0,1 Gew.-%, bezogen auf die gesamte Mischung, eingesetzt. Der Gesamtanteil der Hilfs- und Zusatzstoffe kann 1 bis 5, vorzugsweise 2 bis 4 Gew.-% - bezogen auf die Mittel - betragen.

Ein weiterer Gegenstand der Erfindung betrifft ferner die Verwendung von wäßrigen Mischungen, enthaltend
(a) Aikyl- und/oder Alkenyloligoglykoside,
(b) Ölkörper und
(c) nichtionische Emulgatoren
zur Herstellung von Deostiften.

### Beispiele

**Beispiele 1 bis 9.** Die Komponenten gemäß Tabelle 1 wurden bei ca. 80°C gemeinsam aufgeschmolzen und homogen verrührt. Anschließend erfolgte das Ausgießen in eine vorgewärmte Stiftform. Die anwendungstechnischen Eigenschaften wurden subjektiv wie folgt bestimmt:

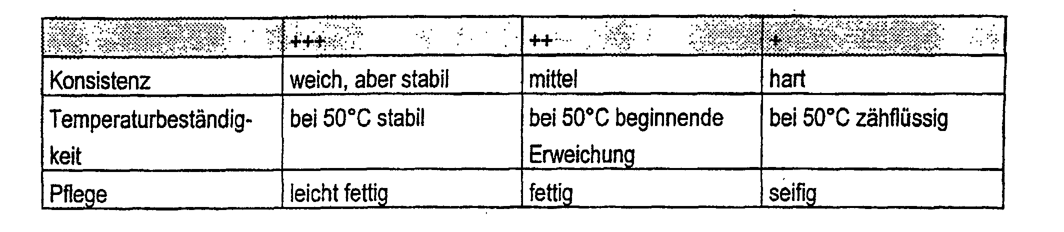

Die Ergebnisse sind in Tabelle 1 zusammengefaßt.

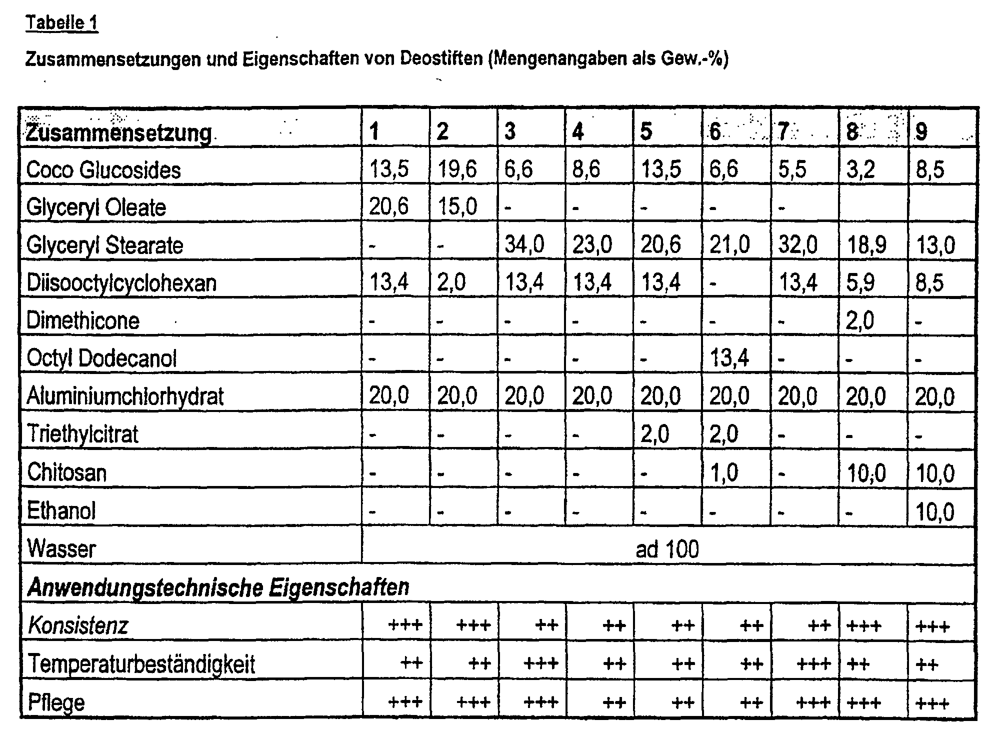

## Patentansprüche

1. Wäßrige kosmetische Zubereitungen in Stiftform, enthaltend
(a) Alkyl- und/oder Alkenyloligoglykoside,
(b) Ölkörper und
(c) nichtionische Emulgatoren,
mit der Maßgabe, daß die Zubereitungen frei von Seifen und natürlichen Wachsen sind.

2. Zubereitungen nach Anspruch 1, **dadurch gekennzeichnet, daß** sie Alkyl- und/oder Alkenyloligoglykoside der Formel **(I)** enthalten,
**R**^{**1**}**O-[G]**_{**p**} (I)
in der R¹ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht.

3. Zubereitungen nach den Ansprüchen 1 und 2, **dadurch gekennzeichnet, daß** sie Ölkörper enthalten, die ausgewählt sind aus der Gruppe, die gebildet wird Guerbetalkoholen auf Basis von Fettalkoholen mit 6 bis 18, vorzugsweise 8 bis 10 Kohlenstoffatomen, Estern von linearen C₆-C₂₀-Fettsäuren mit linearen C₆-C₂₀-Fettalkoholen, Estem von verzweigten C₆-C₁₃-Carbonsäuren mit linearen C₆-C₂₀-Fettalkoholen, Estem von linearen C₆-C₁₈-Fettsäuren mit verzweigten Alkoholen, Estern von linearen und/oder verzweigten Fettsäuren mit mehrwertigen Alkoholen und/oder Guerbetalkoholen, Triglyceriden auf Basis C₆-C₁₀-Fettsäuren, Estem von C₆-C₂₂-Fettalkoholen und/oder Guerbetalkoholen mit aromatischen Carbonsäuren, Dicarbonsäureestem, pflanzlichen Ölen, verzweigten primären Alkoholen, substituierten Cyclohexanen, linearen C₆-C₂₂-Fettalkoholcarbonaten, Guerbetcarbonaten, Dialkylethen, Ringöffnungsprodukten von epoxidierten Fettsäureestern mit Polyolen, Siliconölen und/oder ali-phatischen bzw. naphthenischen Kohlenwasserstoffen.

4. Zubereitungen nach den Ansprüchen 1 bis 3, **dadurch gekennzeichnet, daß** sie nichtionische Emulgatoren enthalten, die ausgewählt sind aus der Gruppe, die gebildet wird von AnlagerungsProdukten von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe; C_{12/18}-Fettsäuremono- und -diestern von Anlagerungsprodukten von 1 bis 30 Mol Ethylenoxid an Glycerin ; Glycerinmono- und -diestem und Sorbitanmono- und -diestern von gesättigten und ungesättigten Fettsäuren mit 6 bis 22 Kohlenstoffatomen und deren Ethylenoxidanlagerungsprodukten; Anlagerungsprodukten von 15 bis 60 Mol Ethylenoxid an Ricinusöl und/oder gehärtetes Ricinusöl; Polyolestern; Anlagerungsprodukten von 2 bis 15 Mol Ethylenoxid an Ricinusöl und/oder gehärtetes Ricinusöl; Partialestem auf Basis linearer, verzweigter, ungesättigter bzw. gesättigter C_{12/22}-Fettsäuren, Ricinolsäure sowie 12-Hydroxystearinsäure und Glycerin, Polyglycerin, Pentaerythrit, Dipentaerythrit, Zuckeralkohole sowie Polyglucoside; Alkylphosphaten; Wollwachsalkoholen; Polysiloxan-Polyalkyl-Polyether-Copolymeren; Mischestem aus Pentaerythrit, Fettsäuren, Citronensäure und Fettalkohol und Polyalkylenglycolen.

5. Zubereitungen nach den Ansprüchen 1 bis 4, **dadurch gekennzeichnet, daß** sie weiterhin als Co-Emulgatoren anionische und/oder amphotere bzw. zwitterionische Tenside enthalten.

6. Zubereitungen nach den Ansprüchen 1 bis 5, **dadurch gekennzeichnet, daß** sie weiterhin als Konsistenzgeber Fettalkohole, Diole und/oder Fettsäurepartialglyceride enthalten.

7. Zubereitungen nach den Ansprüchen 1 bis 6, **dadurch gekennzeichnet, daß** sie weiterhin Wirkstoffe enthalten, die ausgewählt sind aus der Gruppe die gebildet wird von Aluminiumchlorhydraten, Trialkylcitraten und Chitosan..

8. Zubereitungen nach den Ansprüchen 1 bis 7, **dadurch gekennzeichnet, daß** sie weiterhin Siliconverbindungen enthalten.

9. Zubereitungen nach den Ansprüchen 1 bis 8, **dadurch gekennzeichnet, daß** sie
(a) 1 bis 40 Gew.-% Alkyl- und/oder Alkenyloligoglykoside,
(b) 1 bis 30 Gew.-% nichtionische Emulgatoren,
(c) 10 bis 50 Gew.-% Ölkörper,
(d) 0 bis 35 Gew.-% Co-Emulgatoren,
(e) 0 bis 35 Gew.-% Konsistenzgeber
(f) 0 bis 30 Gew.-% Wirkstoffe
(g) 0 bis 20 Gew.-% Siliconverbindungen
mit der Maßgabe enthalten, daß sich die Angaben mit Wasser und weiteren üblichen Hilfs- und Zusatzstoffen zu 100 Gew.-% addieren.

10. Verwendung von Mischungen, enthaltend (a) Alkyl- und/oder Alkenyloligoglykoside,(b) Ölkörper und (b) nichtionische Emulgatoren zur Herstellung von seifen- und wachsfreien Deostiften.

## Claims

1. Water-based cosmetic preparations in stick form containing
(a) alkyl and/or alkenyl oligoglycosides,
(b) oil components and
(c) nonionic emulsifiers,
with the proviso that the preparations are free from soaps and natural waxes.

2. Preparations as claimed in claim 1, **characterized in that** they contain alkyl and/or alkenyl oligoglycosides corresponding to formula **(I)**:
**R**^{**1**}**O-[G]**_{**p**} (I)
in which R¹ is an alkyl and/or alkenyl group containing 4 to 22 carbon atoms, G is a sugar unit containing 5 or 6 carbon atoms and p is a number of 1 to 10.

3. Preparations as claimed in claims 1 and 2, **characterized in that** they contain oil components selected from the group consisting of Guerbet alcohols based on fatty alcohols containing 6 to 18 and preferably 8 to 10 carbon atoms, esters of linear C₆₋₂₀ fatty acids with linear C₆₋₂₀ fatty alcohols, esters of branched C_{6ν13} carboxylic acids with linear C₆₋₂₀ fatty alcohols, esters of linear C₆₋₁₈ fatty acids with branched alcohols, esters of linear and/or branched fatty acids with polyhydric alcohols and/or Guerbet alcohols, triglycerides based on C₆₋₁₀ fatty acids, esters of C₆₋₂₂ fatty alcohols and/or Guerbet alcohols with aromatic carboxylic acids, dicarboxylic acid esters, vegetable oils, branched primary alcohols, substituted cyclohexanes, linear C₆₋₂₂ fatty alcohol carbonates, Guerbet carbonates, dialkyl ethers, ring opening products of epoxidized fatty acid esters with polyols, silicone oils and/or aliphatic or naphthenic hydrocarbons.

4. Preparations as claimed in claims 1 to 3, **characterized in that** they contain nonionic emulsifiers selected from the group consisting of products of the addition of 2 to 30 moles of ethylene oxide and/or 0 to 5 moles of propylene oxide onto linear C₈₋₂₂ fatty alcohols, C₁₂₋₂₂ fatty acids and alkyl phenols containing 8 to 15 carbon atoms in the alkyl group; C_{12/18} fatty acid monoesters and diesters of addition products of 1 to 30 moles of ethylene oxide onto glycerol; glycerol mono- and diesters and sorbitan mono- and diesters of saturated and unsaturated C₆₋₂₂ fatty acids and ethylene oxide addition products thereof; addition products of 15 to 60 moles of ethylene oxide onto castor oil and/or hydrogenated castor oil; polyol esters; addition products of 2 to 15 moles of ethylene oxide onto castor oil and/or hydrogenated castor oil; partial esters based on linear, branched, unsaturated or saturated C_{12/22} fatty acids, ricinoleic acid and 12-hydroxystearic acid and glycerol, polyglycerol, pentaerythritol, dipentaerythritol, sugar alcohols and polyglucosides; alkyl phosphates; wool wax alcohols; polysiloxane/polyalkyl/polyether copolymers; mixed esters of pentaerythritol, citric acid and fatty alcohol and polyalkylene glycols.

5. Preparations as claimed in claims 1 to 4, **characterized in that** they additionally contain anionic and/or amphoteric or zwitterionic surfactants as co-emulsifiers.

6. Preparations as claimed in claims 1 to 5, **characterized in that** they additionally contain fatty alcohols, diols and/or fatty acid partial glycerides as consistency factors.

7. Preparations as claimed in claims 1 to6 , **characterized in that** they additionally contain active substances selected from the group consisting of aluminium chlorohydrates, trialkyl citrates and chitosan.

8. Preparations as claimed in claims 1 to 7, **characterized in that** they additionally contain silicone compounds.

9. Preparations as claimed in claims 1 to 8, **characterized in that** they contain
(a) 1 to 40% by weight of alkyl and/or alkenyl oligoglycosides,
(b) 1 to 30% by weight of nonionic emulsifiers,
(c) 10 to 50% by weight of oil components,
(d) 0 to 35% by weight of co-emulsifiers,
(e) 0 to 35% by weight of consistency factors,
(f) 0 to 30% by weight of active substances,
(g) 0 to 20% by weight of silicone compounds,
with the proviso that the quantities shown add up to 100% by weight with water and other typical auxiliaries and additives.

10. The use of mixtures containing (a) alkyl and/or alkenyl oligoglycosides, (b) oil components and (b) nonionic emulsifiers for the production of soap- and wax-free deodorant sticks.

## Revendications

1. Préparations cosmétiques aqueuses sous forme de bâtonnet, contenant
(a) des alkyl- et alcényloligoglycosides,
(b) des corps huileux et
(c) des émulsifiants non ioniques
sous réserve que les préparations sont dépourvues de savons et de cires naturelles.

2. Préparations selon la revendication 1,
**caractérisées en ce qu'**
elles contiennent des alkyl- et/ou alcényl oligoglycosides de formule (I)
R¹O-[G]ₚ (I)
dans laquelle R¹ représente un radical allyle et/ou alcényle comportant de 4 à 22 atomes de carbone, G représente un radical sucré comportant 5 à 6 atomes de carbone et p représente des nombres allant de 1 à 10.

3. Préparations selon les revendications 1 et 2,
**caractérisées en ce qu'**
elles contiennent des corps huileux qui sont choisis dans le groupe formé par les alcools de Guerbet à base d'alcools gras comportant de 6 à 18, de préférence de 8 à 10 atomes de carbone, les esters d'acides gras en C₆ à C₂₀ linéaires avec des alcools gras en C₆ à C₂₀ linéaires, les esters d'acides carboayliques en C₆ à C₁₃ ramifiés avec des alcools gras en C₆ à C₂₀ linéaires, les esters d'acides gras en C₆ à C₁₈ linéaires avec des alcools ramifiés, les esters d'acides gras linéaires et/ou ramifiés avec des alcools polyvalents et/ou des alcools de Guerbet, les triglycérides à base d'acides gras en C₆ à C₁₀, les esters d'alcools gras en Ce à C₂₂ et/ou les alcools de Guerbet avec des acides carboxyliques aromatiques, des esters d'acide dicarboxylique, les huiles végétales, les alcools primaires ramifiés, les cyclohexanes substitués, les carbonates d'alcool gras en C₆ à C₂₂ linéaires, les carbonates de Guerbet, les éthers dialkyle, les produits d'ouverture de cycle d'esters d'acide gras époxydé avec des polyols, des huiles de silicone et/ou des hydrocarbures aliphatiques ou selon les cas naphténiques.

4. Préparations selon les revendications 1 à 3,
**caractérisées en ce qu'**
elles contiennent des émulsifiants non toniques qui sont choisis dans le groupe formé par les produits de fixation de 2 à 30 moles d'oxyde d'éthylène et/ou de 0 à 5 moles d'oxyde de propylène sur des alcools gras linéaires comportant de 8 à 22 atomes de carbone, sur des acides gras comportant de 12 à 22 atomes de carbone et sur des alkylphénols comportant de 8 à 15 atomes de carbone dans le groupe alkyle ; des mono- et diesters d'acide gras en C₁₂ à C₁₈ de produits de fixation de 1 à 30 moles d'oxyde d'éthylène sur la glycérine ; les mono- et les diesters de glycérine, et les mono- et diesters de sorbitane d'acides gras saturés et insaturés comportant de 6 à 22 atomes de carbone et leurs produits d'addition sur l'oxyde d'éthylène ; les produits de fixation de 15 à 60 moles d'oxyde d'éthylène sur l'huile de ricin et/ou l'huile de ricin durcie ; les éthers de polyol ; les produits de fixation de 2 à 15 moles d'oxyde d'éthylène sur l'huile de ricin et/ou l'huile de ricin durcie ; les esters partiels à base d'acides gras en C₁₂ à C₂₂ linéaires, ramifiés, insaturés ou selon les cas saturés, l'acide ricinoléique ainsi que l'acide 12-hydroxystéarique et la glycérine ; la polyglycérine, le pentaérythritol, le dipentaérythritol, les alcools sacchariques ainsi que les polyglucosides ; et les phosphates d'alkyle ; les alcools de lanoline ; les copolymères polysiloxane-polyalkyl-polyéther ; les esters mixtes de pentaérythritol, les acides gras, l'acide citrique et l'alcool gras ainsi que les polyalkylène glycols.

5. Préparations selon les revendications 1 à 4,
**caractérisées en ce qu'**
elles contiennent en outre comme coémulsifiants des agents tensioactifs anioniques et/ou amphotères ou selon les cas hermaphrodites.

6. Préparations selon les revendications 1 à 5,
**caractérisées en ce qu'**
elles contiennent en outre comme agents donneurs de consistance des alcools gras, des diols et/ou des glycérides partiels d'acide gras.

7. Préparations selon les revendications 1 à 6,
**caractérisées en ce qu'**
elles contiennent en outre des substances actives qui sont choisies dans le groupe formé par les chlorhydrates d'aluminium, les citrates de trialkyle et le chitosane.

8. Préparations selon les revendications 1 à 7,
**caractérisées en ce qu'**
elles contiennent en outre des composés de silicium.

9. Préparations selon les revendications 1 à 8,
**caractérisées en ce qu'**
elles contiennent
(a) de 1 à 40 en poids d'alkyl- et/ou alcényloligoglycoside,
(b) de 10 à 50 en poids d'émulsifiant non ionique,
(c) de 10 à 50 % en poids de corps huileux,
(d) de 0 à 35 % en poids de coémulsiflants,
(e) de 0 à 35 % en poids d'agents donneurs de consistance
(f) de 0 à 30 % en poids de substance active,
(g) de 0 à 20 % en poids de composés de silicium,
sous réserve que les indications se complètent à 100 % avec de l'eau et le cas échéant d'autres adjuvants et additifs habituels.

10. Utilisation de mélanges contenant (a) des alkyl- et/ou alcényloligoglycosides, (b) des corps huileux et (b) des émulsifiants non ioniques pour la fabrication de bâtonnets déodorants dépourvus de savons et de cires.
